(19) Europäisches Patentamt
European Patent Office
Office européen des brevets

(11) EP 4 289 895 A1

(12) EUROPEAN PATENT APPLICATION
published in accordance with Art. 153(4) EPC

(43) Date of publication:
**13.12.2023 Bulletin 2023/50**

(21) Application number: **22887665.2**

(22) Date of filing: **27.10.2022**

(51) International Patent Classification (IPC):
***C08K 5/092** (2006.01)* ***C08K 5/00** (2006.01)*
***C08L 101/00** (2006.01)*

(52) Cooperative Patent Classification (CPC):
**C08K 5/00; C08K 5/092; C08L 101/00**

(86) International application number:
**PCT/KR2022/016615**

(87) International publication number:
**WO 2023/075470 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.10.2021 KR 20210146515**

(71) Applicant: **LG Chem, Ltd.**
**Seoul 07336 (KR)**

(72) Inventors:
- **KIM, Hyun Kyu**
**Daejeon 34122 (KR)**
- **JEONG, Seok Ho**
**Daejeon 34122 (KR)**
- **KIM, Eun Suk**
**Daejeon 34122 (KR)**
- **KIM, Joo Ho**
**Daejeon 34122 (KR)**
- **WOO, Seung Taek**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

# (54) PROPANE TRICARBOXYLATE-BASED PLASTICIZER COMPOSITION, AND RESIN COMPOSITION COMPRISING SAME

(57) The present invention is characterized by propane tricarboxylate, wherein an alkyl group is derived from an isomer mixture of hexyl alcohol, and when the propane tricarboxylate is applied to a resin, excellent tensile properties, elongation properties, migration resistance, volatile loss, and stress resistance as well as high plasticization efficiency may be obtained.

EP 4 289 895 A1

## Description

### TECHNICAL FIELD

The present invention relates to a plasticizer composition including propane tricarboxylate having an alkyl group of various structures in the same number of carbon atoms, and a resin composition including the same.

### BACKGROUND ART

Typically, with respect to a plasticizer, alcohol reacts with polycarboxylic acid such as phthalic acid and adipic acid to form ester corresponding thereto. In addition, in consideration of domestic and foreign regulations limiting phthalate-based plasticizers that are harmful to human body, research into plasticizer compositions, which may replace phthalate-based plasticizers such as terephthalate-based plasticizers, adipate-based plasticizers, and other polymer-based plasticizers, has continued.

Meanwhile, there is a growing demand for eco-friendly products in a plastisol industry such as flooring, wallpaper, and soft and hard sheets, a calendaring industry, and extrusion/injection compound industries, and in an attempt to enhance quality characteristics, processability, and productivity of each end product in response, it is necessary to use a suitable plasticizer in consideration of discoloration, migration properties, mechanical properties, and the like.

Supplementary materials such as a plasticizer, a filler, a stabilizer, a viscosity reducing agent, a dispersant, an antifoaming agent, and a foaming agent are mixed with a PVC resin according to properties required by industry in various areas of use, for example, tensile strength, elongation rate, light resistance, migration properties, gelling properties, absorption rate, or the like.

For example, among the plasticizer compositions applicable to PVC, when di(2-ethylhexyl) terephthalate, which is most commonly used at relatively low cost, is applied, hardness or sol viscosity is high, the absorption rate of the plasticizer is relatively slow, and migration properties and stress migration properties are poor.

In an effort to overcome the limitations described above, as a composition including DEHTP, a product obtained from transesterification with butanol may be considered as an option for a plasticizer. In this case, using the product increases plasticization efficiency, but results in poor volatile loss or thermal stability, and slightly degraded mechanical properties, requiring an improvement in physical properties. Accordingly, there is currently no solution other than to use a secondary plasticizer together in general to make up for the drawbacks.

However, the use of a secondary plasticizer brings about drawbacks of generating unexpected defects as follows: changes in physical properties are hard to predict, unit cost of products may increase, improvements in physical properties are not apparent except for certain cases, and compatibility with a resin may be in trouble.

In addition, when a trimellitate-based material such as tri(2-ethylhexyl) trimellitate or triisononyl trimellitate is used to improve the poor migration properties and loss properties of the DEHTP products, the migration or loss properties may be improved, but plasticization efficiency may be degraded, and a considerable amount of the material is required to be added to provide a resin with proper plasticizing properties, and the relatively high unit price of the products prevents the products from being made commercially available.

Accordingly, there is a need to develop a product for resolving environmental issues caused by phthalate-based products as a typical product, or a product in which the poor physical properties of eco-friendly products are corrected to address the environmental issues of the phthalate-based products.

### DISCLOSURE OF THE INVENTION

### TECHNICAL PROBLEM

An aspect of the present invention provides a plasticizer composition capable of improving migration and plasticization efficiency and also maintaining mechanical properties and stress resistance to at least the same level to those of typical plasticizers by including propane tricarboxylate in which an alkyl group is derived from an isomer mixture of hexyl alcohol.

### TECHNICAL SOLUTION

In order to address the above-described tasks, the present invention provides a plasticizer composition and a resin composition including the plasticizer composition.

(1) According to an aspect of the present invention, in particular, there is provided a tricarboxylate-based plasticizer composition including a propane tricarboxylate-based composition containing at least one propane tricarboxylate

of Formula 1 below, an alkyl group of the propane tricarboxylate is derived from an isomer mixture of hexyl alcohol, and the isomer mixture of hexyl alcohol includes two or more selected from the group consisting of 1-hexanol, 1-methylpentanol, 2-methylpentanol, 3-methylpentanol, 4-methylpentanol, 1,1-dimethylbutanol, 1,2-dimethylbutanol, 1,3-dimethylbutanol, 2,2-dimethylbutanol, 2,3-dimethylbutanol, 3,3-dimethylbutanol, 1-ethylbutanol, 2-ethylbutanol, 3-ethylbutanol, and cyclopentylmethanol.

[Formula 1]

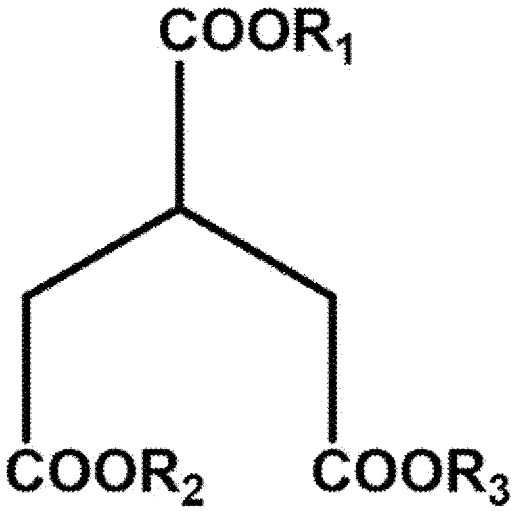

In Formula 1 above,
$R_1$ to $R_3$ are each independently an n-hexyl group, a branched hexyl group, or a cyclopentylmethyl group.

(2) The present invention provides the plasticizer composition according to (1) above, wherein the isomer mixture of hexyl alcohol has a degree of branching of 2.0 or less.
(3) The present invention provides the plasticizer composition according to (1) or (2) above, wherein the isomer mixture of hexyl alcohol has a degree of branching of 1.5 or less.
(4) The present invention provides the plasticizer composition according to any one of (1) to (3) above, wherein the isomer mixture of hexyl alcohol includes 1-hexanol, 2-methylpentanol, and 3-methylpentanol.
(5) The present invention provides the plasticizer composition according to any one of (1) to (4) above, wherein the isomer mixture of hexyl alcohol includes a branched alcohol in an amount of 40 parts by weight or more with respect to 100 parts by weight of the isomer mixture.
(6) The present invention provides the plasticizer composition according to any one of (1) to (5) above, wherein the isomer mixture of hexyl alcohol includes a branched alcohol in an amount of 50 parts by weight to 95 parts by weight with respect to 100 parts by weight of the isomer mixture.
(7) The present invention provides the plasticizer composition according to any one of (1) to (6) above, wherein the isomer mixture of hexyl alcohol includes 1-hexanol in an amount of 40 parts by weight or less with respect to 100 parts by weight of the isomer mixture.
(8) The present invention provides the plasticizer composition according to any one of (1) to (7) above, wherein the isomer mixture of hexyl alcohol includes 1-hexanol, 2-methylpentanol, 3-methylpentanol, and cyclopentylmethanol.
(9) The present invention provides the plasticizer composition according to any one of (1) to (8) above, wherein the isomer mixture of hexyl alcohol includes cyclopentylmethanol in an amount of 20 parts by weight or less with respect to 100 parts by weight of the isomer mixture.
(10) According to another aspect of the present invention, there is provided a resin composition including a resin in an amount of 100 parts by weight, and the plasticizer composition according to any one of (1) to (9) above in an amount of 5 parts by weight to 150 parts by weight.
(11) The present invention provides the resin composition according to (10) above, wherein the resin is one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, polylactic acid, natural rubber, and synthetic rubber.

## ADVANTAGEOUS EFFECTS

A plasticizer composition according to an embodiment of the present invention, when used in a resin composition, may maintain and improve mechanical properties and stress resistance to at least the same level to those of typical plasticizers, and also improve properties such as loss properties and plasticization efficiency.

## MODE FOR CARRYING OUT THE INVENTION

It will be understood that words or terms used in the specification and claims of the present invention shall not be construed as being limited to having the meaning defined in commonly used dictionaries. It will be further understood that the words or terms should be interpreted as having meanings that are consistent with their meanings in the context of the relevant art and the technical idea of the invention, based on the principle that an inventor may properly define the meaning of the words or terms to best explain the invention.

### Definition of Terms

As used herein, the term "composition" encompasses a mixture of materials including the composition as well as reaction products and decomposition products formed from materials of the composition.

As used herein, the term "straight vinyl chloride polymer" is one kind of vinyl chloride polymer, which is polymerized through suspension polymerization, bulk polymerization, or the like, and refers to a polymer which is in the form of a porous particle in which a large amount of pores with a size of several tens to several hundreds of micrometers are distributed and has no cohesion and excellent flowability.

As used herein, the term "paste vinyl chloride polymer" is one kind of vinyl chloride polymer, which is polymerized through microsuspension polymerization, microseed polymerization, emulsion polymerization, or the like, and refers to a polymer which is in the form of a fine, compact, and non-porous particle with a size of several tens to several thousands of nanometers and has cohesion and poor flowability.

The terms "including" and "having", and derivatives thereof are not intended to exclude the presence of any additional components, steps, or procedures, whether they are specifically disclosed or not. To avoid any uncertainty, all compositions claimed through the use of the term "including", whether polymers or otherwise, may include any additional additives, adjuvants, or compounds unless otherwise stated. In contrast, the term "consisting essentially of" excludes any other component, step, or procedure from the scope of any subsequent description, and excludes those that are not essential to operability. The terms "consisting of" excludes any component, step, or procedure that is not specifically described or listed.

### Measurement Methods

In the specification, the contents of components in the composition are analyzed through gas chromatography analysis using a gas chromatography instrument (Agilent 7890 GC manufactured by Agilent Technologies Inc., column: HP-5, carrier gas: helium (flow rate 2.4 mL/min), detector: F.I.D, injection volume: 1 μL, initial value: 70 °C/4.2 min, terminal value: 280 °C/7.8 min, program rate: 15 °C/min).

In the specification, "hardness"' refers to Shore hardness (Shore "A" and/or Shore "D") as measured at 25 °C in accordance with ASTM D2240. Hardness is measured under the conditions of 3T 10s and may be an index for evaluating plasticization efficiency, and low hardness indicates excellent plasticization efficiency.

In the specification, "tensile strength" is measured in accordance with ASTM D638 as follows. A 1T specimen is pulled at a cross head speed of 200 mm/min using a universal testing machine (UTM; 4466 manufactured by Instron), a point where the specimen is broken is then determined and substituted into the following Equation 1.

[Equation 1]

$$\text{Tensile strength (kgf/cm}^2\text{)} = \text{load value (kgf)} / \text{thickness (cm)} \times \text{width (cm)}$$

In the specification, "elongation rate" is measured in accordance with ASTM D638 as follows. A 1T specimen is pulled at a cross head speed of 200 mm/min using the UTM, a time point at which the specimen is broken is then determined and substituted into the following Equation 2.

[Equation 2]

Elongation rate (%)=length after elongation/initial length×100

In the specification, "migration loss" is measured in accordance with KSM-3156 as follows. A specimen with a thickness of 2 mm or more is prepared, glass plates are attached to both sides of the specimen, and a load of 1 $kgf/cm^2$ is then applied. The specimen is placed in a hot-air convection oven (80 °C) for 72 hours, then taken out of the oven, and cooled at room temperature for 4 hours. Afterward, the glass plates attached to both sides of the specimen are removed, weights of the specimen before being placed in and after taken out of the oven along with the glass plate were measured to determine migration loss through the following Equation 3.

[Equation 3]

Migration loss (%)={(initial weight of specimen-weight of specimen after being taken out of oven)/initial weight of specimen }×100

In the specification, "volatile loss" is measured by processing a specimen at 80 °C for 72 hours and then weighing the specimen.

[Equation 4]

Volatile loss (wt%)={(initial weight of specimen-weight of specimen after being processed)/initial weight of specimen}×100

In the case of the above-described various measurement methods, detailed conditions such as temperature, rotation speed, time, and the like may be slightly varied in some cases, and when the detailed conditions are varied, the measurement method and conditions are separately specified.

Hereinafter, the present invention will be described in detail to aid in understanding of the present invention.

According to an embodiment of the present invention, there is provided a tricarboxylate-based plasticizer composition including a propane tricarboxylate-based composition containing at least one propane tricarboxylate of Formula 1 below, an alkyl group of the propane tricarboxylate is derived from an isomer mixture of hexyl alcohol, and the isomer mixture of hexyl alcohol includes two or more selected from the group consisting of 1-hexanol, 1-methylpentanol, 2-methylpentanol, 3-methylpentanol, 4-methylpentanol, 1,1-dimethylbutanol, 1,2-dimethylbutanol, 1,3-dimethylbutanol, 2,2-dimethylbutanol, 2,3-dimethylbutanol, 3,3-dimethylbutanol, 1-ethylbutanol, 2-ethylbutanol, 3-ethylbutanol, and cyclopentylmethanol.

[Formula 1]

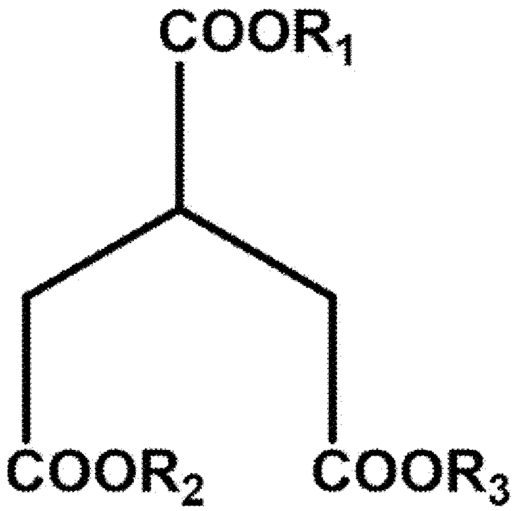

In Formula 1,

$R_1$ to $R_3$ are each independently an n-hexyl group, a branched hexyl group, or a cyclopentylmethyl group.

The plasticizer composition may be a product generated by a direct esterification between 1,2,3-propane tricarboxylic acid or a derivative thereof and a hexyl alcohol mixture, or a transesterification between a trialkyl 1,2,3-tricarboxylate and a hexyl alcohol mixture, and the hexyl alcohol mixture applied in this case may be a mixture of hexyl alcohols having different structures, that is, a mixture of structural isomers. To be more specific, the isomer mixture of hexyl alcohol may include two or more selected from the group consisting of 1-hexanol, 1-methylpentanol, 2-methylpentanol, 3-methylpentanol, 4-methylpentanol, 1,1-dimethylbutanol, 1,2-dimethylbutanol, 1,3-dimethylbutanol, 2,2-dimethylbutanol, 2,3-dimethylbutanol, 3,3-dimethylbutanol, 1-ethylbutanol, 2-ethylbutanol, 3-ethylbutanol, and cyclopentylmethanol.

The alkyl groups of $R_1$ to $R_3$ of Formula 1 above may be determined based on the alcohol included in such a hexyl alcohol isomer, and in a final composition, diverse compositions in which three, two, or one of the isomer alkyl groups of hexyl alcohol are bonded to three alkyl groups may be included, and a ratio of components in the final composition may be determined by the ratio of respective structural isomer components in a hexyl alcohol mixture being reacted.

As described above, with respect to applying a tricarboxylate-based plasticizer, when an alcohol having 6 carbon atoms is used, excellent mechanical properties may be provided, and more specifically, tensile strength, elongation rate, and volatile loss may be markedly improved compared to an alcohol having less than 6 carbon atoms, and excellent plasticization efficiency, migration resistance, and stress resistance may be achieved compared to an alcohol having greater than 6 carbon atoms.

The isomer mixture of hexyl alcohol of the plasticizer composition according to an embodiment of the present invention has a degree of branching of 2.0 or less, preferably, 1.5 or less. Specifically, the degree of branching may be 1.5 or less, 1.3 or less, more preferably, 1.1 or less, particularly preferably 1.0 or less. In addition, the degree of branching may be 0.1 or more, 0.2 or more, 0.3 or more, most preferably, 0.7 or more. The degree of branching of the isomer mixture of hexyl alcohol may also remain the same in the tricarboxylate-based plasticizer composition produced from hexyl alcohol. When the degree of branching is too high, balance between physical properties may be broken to prevent products from meeting any one or more evaluation standards, but in a preferred range of 1.5 or less, the improvement of migration loss and volatile loss as well as mechanical properties may be even further optimized, and an excellent balance between physical properties may be obtained.

In this case, the degree of branching may indicate the number of branched carbons of alkyl groups bonded to a material included in a composition, and may be determined according to a weight ratio of a corresponding material. For example, when an alcohol mixture includes 60 wt% of n-hexyl alcohol, 30 wt% of methylpentyl alcohol, and 10 wt% of ethylbutyl alcohol, the number of branched carbons of each alcohol is 0, 1, and 2, and the degree of branching may be 0.5 as determined by $[(60\times0)+(30\times1)+(10\times2)]/100$. Meanwhile, with respect to defining the degree of branching in the present invention, the number of branched carbons of cyclopentylmethanol is regarded as 0.

The isomer mixture of hexyl alcohol may include 1-hexanol and 2-methylpentanol. As such, when 1-hexanol and 2-methylpentanol are included together, balance between physical properties may be maintained, and excellent

effects with regard to volatile loss may be achieved. Preferably, the isomer mixture of hexyl alcohol may further include 3-methylpentanol, and in this case, the balance between physical properties is fairly great.

A branched hexyl alcohol including the 2-methylpentanol may be included in an amount of 40 parts by weight or more, 50 parts by weight or more, 60 parts by weight or more, preferably, 65 parts by weight or more, 70 parts by weight or more, with respect to 100 parts by weight of the isomer mixture. The branched hexyl alcohol including the 2-methylpentanol may be included in an amount of 100 parts by weight or less, 99 parts by weight or less, 98 parts by weight or less, preferably, 95 parts by weight or less, or 90 parts by weight or less. When the branched hexyl alcohol is included in this range, an improvement of mechanical properties may be expected.

In addition, linear alcohol of the 1-hexanol may be included in an amount of 50 parts by weight or less, 40 parts by weight or less, preferably, 30 parts by weight or less, with respect to 100 parts by weight of the isomer mixture. The 1-hexanol may not be present in components, but may be included in an amount of at least 2 parts by weight or more, and in this case, balance between physical properties is maintained and also improving mechanical properties is achieved. Theoretically, linear alcohol is known to exhibit excellent properties, but in the present invention, different results were obtained, and it was confirmed that even better balance between physical properties was obtained using an isomer mixture including branched alcohol.

In addition, the isomer mixture of hexyl alcohol may include 1-hexanol, 2-methylpentanol, 3-methylpentanol, and cyclopentylmethanol. Preferably, by further including cyclopentylmethanol, balance between physical properties may be maintained and also mechanical properties and plasticization efficiency may by slightly improved.

In this case, the cyclopentylmethanol may be included in an amount of 20 parts by weight or less, preferably, 15 parts by weight or less, or may not be present with respect to 100 parts by weight of the isomer mixture, or at a minimum of 2 parts by weight to obtain effects thereby.

Specifically, depending on the proportion of the branched alkyl groups among the total alkyl radicals in a final composition, and further, depending on the proportion of a specific branch alkyl radical among the branch type alkyl groups, balance between plasticization efficiency and physical properties, such as migration/loss properties, may be controlled, mechanical properties, such as tensile strength and elongation rate, and stress resistance may be maintained to at least the same level, and this may be achieved from the components of the isomer of hexyl alcohol described above and the component ratios thereof.

Through the way described above, products which may prevent environmental issues of typical phthalate-based products and further improve loss properties may be accomplished, the migration and loss properties of the typical terephthalate-based products may be markedly improved, and products having greatly improved light resistance when compared with the commercially available typical products may be achieved.

As a method of preparing the plasticizer composition according to an embodiment of the present invention, any methods of preparing the above-described plasticizer composition, well-known in the art may be applied without specific limitation.

For example, the composition may be prepared through a direct esterification between 1,2,3-propane tricarboxylic acid or an anhydride thereof and an isomer mixture of hexyl alcohol, or through a transesterification between trialkyl 1,2,3-propane tricarboxylate and an isomer mixture of hexyl alcohol.

The plasticizer composition according to an embodiment of the present invention is a material prepared by suitably performing the esterification, and the preparation method is not particularly limited as long as the above conditions are met and a weight ratio between tricarboxylates for each family is suitably regulated.

For example, the direct esterification may be performed as follows: injecting 1,2,3-propane tricarboxylic acid or a derivative thereof and an isomer mixture of hexyl alcohol, and then adding a catalyst to induce reaction in a nitrogen atmosphere; removing an unreacted raw material; neutralizing (or deactivating) the unreacted raw material and the catalyst; and removing (e.g., using distillation under reduced pressure) and filtering impurities.

Components of the isomer mixture of hexyl alcohol and a weight ratio of the components are the same as described above. The isomer mixture of hexyl alcohol may be used in a range of 200 to 900 mol%, 200 to 700 mol%, 200 to 600 mol%, 250 to 600 mol%, or 270 to 600 mol%, with respect to 100 mol% of acid, and by controlling the amount of the alcohol, the component ratio in a final composition may be controlled.

The catalyst may be, for example, one or more selected from an acid catalyst such as sulfuric acid, hydrochloric acid, phosphoric acid, nitric acid, paratoluenesulfonic acid, methanesulfonic acid, ethanesulfonic acid, propanesulfonic acid, butanesulfonic acid, and alkyl sulfate, metal salt such as aluminum lactate, lithium fluoride, potassium chloride, cesium chloride, calcium chloride, iron chloride, and aluminum phosphate, metal oxide such as heteropoly acid, natural/synthetic zeolite, cation and anion exchange resins, and an organometal such as tetra alkyl titanate and a polymer thereof. Specifically, the catalyst may use, for example, tetra alkyl titanate. Preferably, as an acid catalyst having low activation temperature, paratoluenesulfonic acid, methanesulfonic acid, and the like may be suitable.

The amount of a catalyst to be used may vary depending on the type thereof, and for example, a homogeneous catalyst may be used in an amount of 0.01 to 5 wt%, 0.01 to 3 wt%, 1 to 5 wt%, or 2 to 4 wt%, with respect to total 100 wt% of reactants, and a heterogeneous catalyst may be used in an amount of 5 to 200 wt%, 5 to 100 wt%, 20 to 200

wt%, or 20 to 150 wt% with respect to a total amount of reactants.

In this case, the reaction temperature may be within a range of 100 to 280 °C, 100 to 250 °C, or 120 to 230 °C.

In another example, the transesterification may be a reaction between trialkyl 1,2,3-propane tricarboxylate and an isomer mixture of hexyl alcohol. In terms of minimizing impurities, an alkyl group of the trialkyl 1,2,3-propane tricarboxylate may preferably be a hexyl group, and in this case, an isomer mixture of hexyl alcohol is set to include a branched alcohol when the hexyl group of tricarboxylate is linear, or an isomer mixture of hexyl alcohol is set to include a linear alcohol when the hexyl group of tricarboxylate is branched, and an alkyl group of finally obtained 1,2,3-propane tricarboxylate may thus include both linear and branched types.

"Transesterification" as used herein indicates a reaction between alcohol and ester as shown in Reaction Formula 1 below to interchange R" of the ester with R' of the alcohol:

[Reaction Formula 1]

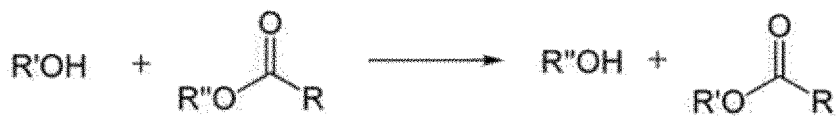

Generally, upon the transesterification, when there are two types of alkyl groups, four types of ester compositions may be produced according to four cases as follows: a case where alkoxide of alcohol attacks carbons of three ester (RCOOR") groups present in an ester compound; a case where alkoxide of alcohol attacks carbons of two ester (RCOOR") groups present in an ester compound; a case where alkoxide of alcohol attacks carbons of one ester (RCOOR") group present in an ester compound; and an unreacted case where no reaction takes place.

However, in tricarboxylate included in the plasticizer composition according to the present invention, when two ester groups are exchanged or one ester group is exchanged according to bonding position of an ester group, three types may be formed for each, and accordingly, a maximum of 8 types of compounds may be present in a final composition. However, in the isomer mixture of hexyl alcohol according to the present invention, two types of alkyl groups are present, and the types may thus be more diverse.

In addition, the transesterification does not cause a wastewater issue unlike acid-alcohol esterification.

A composition ratio of the mixture prepared through the transesterification may be controlled according to an addition amount of alcohol. The addition amount of alcohol may be 0.1 to 200 parts by weight, specifically 1 to 150 parts by weight, more specifically 5 to 100 parts by weight, with respect to 100 parts by weight of the trialkyl 1,2,3-propane tricarboxylate compound. For reference, the component ratio in a final composition may be determined by the addition amount of alcohol as in the direct esterification.

According to an embodiment of the present invention, the transesterification may be performed at a reaction temperature of 120 °C to 190 °C, preferably 135 °C to 180 °C, more preferably 141 °C to 179 °C for 10 minutes to 10 hours, preferably 30 minutes to 8 hours, more preferably 1 hour to 6 hours. The composition ratio of a final plasticizer composition may be efficiently controlled when the temperature and time are within the above ranges. In this case, the reaction time may be determined from a point of reaching the reaction temperature after elevating the temperature of reactants.

The transesterification may be performed in the presence of an acid catalyst or a metal catalyst, and in this case, the reaction time may be reduced.

The acid catalyst may include, for example, sulfuric acid, methanesulfonic acid, or p-toluenesulfonic acid, and the metal catalyst may include, for example, an organometal catalyst, a metal oxide catalyst, a metal salt catalyst, or a metal itself.

The metal component may be, for example, any one selected from the group consisting of tin, titanium, and zirconium, or a mixture of two or more thereof.

In addition, removing unreacted alcohol and reaction by-products through distillation may be further included after the transesterification. The distillation may be, for example, a two-stage distillation by which the alcohol and the by-products are individually separated using a difference in boiling points. In another example, the distillation may be a mixed distillation, In this case, a relatively stable ester-based plasticizer composition having a desired composition ratio may be obtained. The mixed distillation indicates distillation of unreacted alcohol and by-products together.

According to another embodiment of the present invention, a resin composition including the plasticizer composition and a resin is provided.

As the resin, resins known in the art may be used. For example, a mixture of one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, polylactic acid, natural rubber, synthetic rubber, and thermoplastic elastomer may be used, without limitation.

The plasticizer composition may be included in an amount of 5 to 150 parts by weight, preferably 5 to 130 parts by weight, or 10 to 120 parts by weight, with respect to 100 parts by weight of the resin.

Generally, a resin product may be prepared from the resin in which a plasticizer composition is used through melt processing or plastisol processing, and the resin obtained through melt processing and the resin obtained through plastisol processing may be produced differently according to each polymerization method.

For example, when the vinyl chloride polymer is used for melt processing, solid phase resin particles having a large average particle diameter are prepared through suspension polymerization or the like and used, and this vinyl chloride polymer is referred to as a straight vinyl chloride polymer, whereas when the vinyl chloride polymer is used for plastisol processing, a sol state resin as minute resin particles are prepared through emulsion polymerization or the like and used, and this vinyl chloride polymer is referred to as a paste vinyl chloride resin.

In this case, in the case of the straight vinyl chloride polymer, a plasticizer may be preferably included in a range of 5 to 80 parts by weight with respect to 100 parts by weight of the polymer, and in the case of the paste vinyl chloride polymer, a plasticizer may be preferably included in a range of 40 to 120 parts by weight with respect to 100 parts by weight of the polymer.

The resin composition may further include a filler. The filler may be included in an amount of 0 to 300 parts by weight, preferably 50 to 200 parts by weight, more preferably 100 to 200 parts by weight, with respect to 100 parts by weight of the resin.

As the filler, fillers well-known in the art may be used and are not specifically limited. For example, the filler may be a mixture of one or more selected from silica, magnesium carbonate, calcium carbonate, hard coal, talc, magnesium hydroxide, titanium dioxide, magnesium oxide, calcium hydroxide, aluminum hydroxide, aluminum silicate, magnesium silicate, and barium sulfate.

In addition, the resin composition may further include other additives such as a stabilizer, when needed. Each of the other additives such as a stabilizer, may be, for example, in an amount of 0 to 20 parts by weight, preferably 1 to 15 parts by weight, with respect to 100 parts by weight of the resin.

The stabilizer may be, for example, a calcium-zinc-based (Ca-Zn-based) stabilizer such as a composite stearate of calcium-zinc or a barium-zinc-based (Ba-Zn-based) stabilizer, but is not particularly limited.

The resin composition may be applied to both melt processing and plastisol processing as described above, and calendaring processing, extrusion processing, or injection processing may be applied to the melt processing, and coating processing or the like may be applied to the plastisol processing.

## Example

Hereinafter, the present invention will be described in detail with reference to Examples. However, Examples according to the present invention may be modified into other various forms, and the scope of the present invention should not be construed as being limited to Examples described below. Examples of the present invention are provided to describe the present invention more fully to those skilled in the art.

### Example 1

To a reactor equipped with a stirrer, a condenser, and a decanter, 299.4 g of 1,2,3-propane tricarboxylic acid, 781.7 g of an isomer mixture of hexyl alcohol, and 0.9 g of tetrabutyl titanate (TnBT) were added, and subjected to esterification in a nitrogen atmosphere. After terminating the reaction, a catalyst and the product thus obtained were neutralized with an aqueous alkali solution, and unreacted alcohol and water were purified to finally obtain a plasticizer composition.

The alcohol composition of the isomer mixture of hexyl alcohol used herein is a mixture of 1-hexanol, 2-methylpentanol, 3-methylpentanol, and cyclopentyl methanol in a weight ratio of 10:40:45:5.

### Example 2

A plasticizer composition was obtained in the same manner as in Example 1 except for using an isomer mixture of hexyl alcohol in which 1-hexanol, 2-methylpentaol, and 3-methylpentanol were mixed in a weight ratio of 20:35:45.

### Example 3

A plasticizer composition was obtained in the same manner as in Example 1 except for using an isomer mixture of hexyl alcohol in which 1-hexanol, 2-methylpentanol, 3-methylpentanol, and cyclopentyl methanol were mixed in a weight ratio of 10:45:30:15.

**Comparative Example 1**

Dioctyl phthalate (DOP, LG Chem) was used as a plasticizer.

**Comparative Example 2**

Diisononyl phthalate (DINP, LG Chem) was used as a plasticizer.

**Comparative Example 3**

Di(2-ethylhexyl) terephthalate (GL300, LG Chem) was used as a plasticizer.

**Comparative Example 4**

A composition (GL500, LG Chem) including 6.3 wt% of dibutyl terephthalate (DBTP), 38.8 wt% of butyl(2-ethylhexyl)terephthalate (BEHTP), and 54.9 wt% of di(2-ethylhexyl)terephthalate (DEHTP) was used as a plasticizer.

**Comparative Example 5**

A plasticizer composition was obtained in the same manner as in Example 1, except that 326.4 g of citric acid was used instead of 1,2,3-propane tricarboxylic acid.

**Comparative Example 6**

A plasticizer composition was obtained in the same manner as in Comparative Example 5, except that after esterification, acetic anhydride was added and subjected to acetylation.

**Comparative Example 7**

A plasticizer composition was obtained in the same manner as in Example 1, except that 781.7 g of a 2-methylpentanol single compound was used instead of an isomer mixture of hexyl alcohol.

**Comparative Example 8**

A plasticizer composition was obtained in the same manner as in Example 1, except that 680 g of a n-pentanol single compound was used instead of an isomer mixture of hexyl alcohol.

**Comparative Example 9**

A plasticizer composition was obtained in the same manner as in Example 1, except that 890 g of a n-heptanol single compound was used instead of an isomer mixture of hexyl alcohol.

The types of acids and alcohols used in Examples and Comparative Examples are shown in Table 1 below.

[Table 1]

| | Acid | Alcohol (parts by weight) | | | |
|---|---|---|---|---|---|
| | | 1-H | 2-MP | 3-MP | CYP-M |
| Example 1 | PTCA | 10 | 40 | 45 | 5 |
| Example 2 | PTCA | 20 | 35 | 45 | 0 |
| Example 3 | PTCA | 10 | 45 | 30 | 15 |
| Comparative Example 1 | DOP | | | | |
| Comparative Example 2 | DINP | | | | |
| Comparative Example 3 | DOTP | | | | |
| Comparative Example 4 | A mixture of DBTP, BOTP, and DOTP (6.3%, 38.8%, 54.9% each) | | | | |

(continued)

| | Acid | Alcohol (parts by weight) | | | |
|---|---|---|---|---|---|
| | | 1-H | 2-MP | 3-MP | CYP-M |
| Comparative Example 5 | CA | 10 | 40 | 45 | 5 |
| Comparative Example 6 | ACA | 10 | 40 | 45 | 5 |
| Comparative Example 7 | PTCA | 0 | 100 | 0 | 0 |
| Comparative Example 8 | PTCA | Using N-pentanol | | | |
| Comparative Example 9 | PTCA | Using n-heptanol | | | |
| PTCA: Propane tricarboxylic acid<br>CA: Citric acid<br>ACA: Acetyl citrate<br>1-H: 1-Hexanol<br>2-MP: 2-Methylpentanol<br>3-MP: 3-Methylpentanol<br>CYP-M: Cyclopentyl methanol | | | | | |

**Experimental Example 1: Sheet Performance Evaluation**

Using the plasticizers of Examples and Comparative Examples, specimens were prepared according to ASTM D638 and prescription and preparation conditions below.

**(1) Prescription:** 100 parts by weight of a straight vinyl chloride polymer (LS100S), 40 parts by weight of a plasticizer, and 3 parts by weight of a stabilizer (BZ-153T)
**(2) Mixing:** mixing at 98 °C at 700 rpm
**(3) Preparation of Specimen:** 1T, 2T, and 3T sheets were prepared by being processed at 160 °C for 4 minutes through a roll mill, and at 180 °C for 2.5 minutes (low pressure) and 2 minutes (high pressure) through a press.
**(4) Evaluation Item**

1) Hardness: Shore hardness (Shore "A" and "D") at 25 °C was determined using a 3T specimen for 10 seconds using ASTM D2240. The plasticization efficiency was determined to be excellent when a value was smaller.
2) Tensile strength and tensile strength retention: Through a method of ASTM D638, a specimen was pulled at a cross head speed of 200 mm/min using a testing machine of U.T.M (manufacturer: Instron, model name: 4466), and a point where the 1T specimen was broken was determined. The tensile strength was determined as follows.

$$\text{Tensile strength (kgf/cm}^2\text{)} = \text{load value (kgf)/thickness (cm)} \times \text{width (cm)}$$

The tensile strength retention is to determine a ratio of tensile strength remaining in a specimen after leaving the specimen in an oven at 100 °C for 168 hours, and the measuring method is the same as the tensile strength measurement method described above.
3) Measurement of tensile elongation and tensile elongation retention: Through a method of ASTM D638, a specimen was pulled at a cross head speed of 200 mm/min using a testing machine of U.T.M, and a point where the 1T specimen was broken was determined.

$$\text{Tensile elongation (\%)} = \text{length after elongation/initial length} \times 100$$

The tensile elongation retention is to determine a ratio of tensile elongation remaining in a specimen after leaving the specimen in an oven at 100 °C for 168 hours, and the measuring method is the same as the tensile elongation

measurement method described above.

4) Measurement of migration loss: According to KSM-3156, a specimen having a thickness of 2 mm or more was obtained, glass plates were attached onto both sides of the 1T specimen, and a load of 1 kgf/cm$^2$ was applied. The specimen is placed in a hot-air convection oven (80 °C) for 72 hours, then taken out of the oven, and cooled at room temperature for 4 hours. Afterward, the glass plates attached to both sides of the specimen were removed, weights of the specimen before being placed in and after taken out of the oven along with the glass plate were measured to determine migration loss through the following Equation.

Migration loss (%)={(initial weight of specimen at room temperature-weight of specimen after placing in oven)/initial weight of specimen at room temperature}×100

5) Measurement of volatile loss: The specimen prepared above was processed at 80 °C for 72 hours, and weight of the specimen was determined.

Volatile loss (wt%)=weight of initial specimen-(weight of specimen after processing at 80 °C for 72 hours)/weight of initial specimen×100

6) Stress test (stress resistance): A specimen having a thickness of 2 mm in a bent state was placed at 23 °C for 168 hours, and the degree of migration (degree of oozing) was observed, and the results were reported as numerical values, and excellent properties were shown as the value was closer to 0.

(5) Evaluation Results

The evaluation results on the items are shown in Table 2 and Table 3 below.

[Table 2]

| | Plasticization efficiency | | Tensile properties | | Elongation properties | |
|---|---|---|---|---|---|---|
| | Shore A | Shore D | Tensil e streng th (kgf/c m$^2$) | Tensile retenti on (%) | Elonga tion rate (%) | Elonga tion retent ion (%) |
| Example 1 | 85.1 | 37.3 | 214.8 | 120.5 | 334.0 | 93.2 |
| Example 2 | 85.3 | 37.5 | 216.7 | 115.9 | 339.8 | 94.5 |
| Example 3 | 85.1 | 37.4 | 224.3 | 118.7 | 345.2 | 96.7 |
| Comparative Example 1 | 87.0 | 40.3 | 212.0 | 123.0 | 311.7 | 82.7 |
| Comparative Example 2 | 89.0 | 42.4 | 225.0 | 100.9 | 319.5 | 93.6 |
| Comparative Example 3 | 90.8 | 43.9 | 243.0 | 99.7 | 334.7 | 92.7 |
| Comparative Example 4 | 87.5 | 41.1 | 233.6 | 124.7 | 325.8 | 84.8 |
| Comparative Example 5 | 86.8 | 39.5 | 204.5 | 105.6 | 311.5 | 89.4 |
| Comparative Example 6 | 90.2 | 43.9 | 235.8 | 101.4 | 311.4 | 91.0 |

(continued)

| | Plasticization efficiency | | Tensile properties | | Elongation properties | |
|---|---|---|---|---|---|---|
| | Shore A | Shore D | Tensil e streng th (kgf/c $m^2$) | Tensile retenti on (%) | Elonga tion rate (%) | Elonga tion retent ion (%) |
| Comparative Example 7 | 85.9 | 37.8 | 201.5 | 104.2 | 310.2 | 92.0 |
| Comparative Example 8 | 83.4 | 36.1 | 200.8 | 84.2 | 275.6 | 65.8 |
| Comparative Example 9 | 88.7 | 41.8 | 210.5 | 108.6 | 321.5 | 95.4 |

[Table 3]

| | Migratio n loss (%) | Volatil e loss (%) | Stress resistance | | |
|---|---|---|---|---|---|
| | | | Day 1 | Day 3 | Day 5 |
| Example 1 | 2.05 | 1.41 | 0.5 | 0.5 | 0 |
| Example 2 | 1.84 | 1.32 | 0.5 | 0 | 0 |
| Example 3 | 1.77 | 1.40 | 0.5 | 0.5 | 0 |
| Comparative Example 1 | 1.49 | 1.86 | 0 | 0 | 0 |
| Comparative Example 2 | 1.94 | 0.85 | 0.5 | 0.5 | 0 |
| Comparative Example 3 | 6.40 | 0.94 | 1 | 2.5 | 3 |
| Comparative Example 4 | 5.17 | 3.21 | 0.5 | 1.5 | 2.5 |
| Comparative Example 5 | 1.64 | 1.38 | 0.5 | 0.5 | 0.5 |
| Comparative Example 6 | 3.28 | 0.96 | 0.5 | 1 | 1 |
| Comparative Example 7 | 1.97 | 1.44 | 0.5 | 0.5 | 0.5 |
| Comparative Example 8 | 1.34 | 5.61 | 0.5 | 0.5 | 1 |
| Comparative Example 9 | 3.68 | 1.10 | 1.5 | 2 | 2 |

Referring to Tables 2 and 3 above, it is seen that Examples 1 to 3 applying the plasticizer composition according to an embodiment of the present invention showed similar levels in terms of migration loss, volatile loss, and stress resistance, and improved plasticization efficiency and elongation rate compared to Comparative Examples 1 and 2 using typical phthalate-based products. Comparative Examples 1 and 2 showed excellent performance, but are typical phthalate-based products that are not eco-friendly, and the above results suggest that the plasticizer composition of the present invention, which is eco-friendly as having no phthalate, may sufficiently replace the typical products.

In addition, Comparative Examples 3 and 4 are eco-friendly products that are used as a replacement for Comparative Examples 1 and 2, but exhibit migration and stress resistance significantly inferior to typical products and Examples 1 to 3 of the present invention, and have poorer plasticization efficiency than Examples 1 to 3 of the present invention.

In addition, in Comparative Examples 5 and 6 where an isomer mixture of hexyl alcohol was applied in the same manner as in Examples 1 to 3 of the present invention, but citric acid or acetyl citric acid was applied instead of 1,2,3-propane tricarboxylic acid, Comparative Example 5 using citric acid showed plasticization efficiency, tensile properties, and elongation properties which are inferior to those of Examples 1 to 3, and Comparative Example 6 using citric acid and further performing acetylation showed plasticization efficiency, elongation properties, migration resistance, and stress resistance which are inferior to those of Examples 1 to 3.

In addition, Comparative Example 7 using 1,2,3-propane tricarboxylic acid in the same manner as in Examples of the present invention, but using a 2-methylpentanol single compound instead of an isomer mixture of hexyl alcohol as alcohol showed tensile properties and elongation properties which are inferior to those of Examples 1 to 3. Comparative

Example 8 using 1,2,3-propane tricarboxylic acid as in Comparative Example 7, but using n-pentanol having 5 carbon atoms alone as alcohol also showed tensile properties and elongation properties which are significantly inferior to those of Examples 1 to 3, and volatile loss and stress resistance which are inferior to those of Examples 1 to 3. Lastly, Comparative Example 9 using n-heptanol of an alcohol having 7 carbon atoms showed plasticization efficiency, tensile properties, elongation properties, migration resistance, and stress resistance which are inferior to those of Examples 1 to 3.

The above results indicate that as in Examples of the present invention, when an esterification product of 1,2,3-propane tricarboxylic acid and an isomer mixture of hexyl alcohol is applied as a plasticizer, a plasticizer that may sufficiently replace typical products and exhibits excellent performance may be achieved and in particular, in the present invention, when acid and alcohol as 1,2,3-propane tricarboxylic acid and an isomer mixture of hexyl alcohol are selected and applied, a plasticizer composition having excellent balance among various physical properties and also excellent physical properties in general may be provided.

## Claims

1. A tricarboxylate-based plasticizer composition comprising a propane tricarboxylate-based composition comprising at least one propane tricarboxylate of Formula 1 below,

   wherein an alkyl group of the propane tricarboxylate is derived from an isomer mixture of hexyl alcohol, and the isomer mixture of hexyl alcohol comprises two or more selected from the group consisting of 1-hexanol, 1-methylpentanol, 2-methylpentanol, 3-methylpentanol, 4-methylpentanol, 1,1-dimethylbutanol, 1,2-dimethylbutanol, 1,3-dimethylbutanol, 2,2-dimethylbutanol, 2,3-dimethylbutanol, 3,3-dimethylbutanol, 1-ethylbutanol, 2-ethylbutanol, 3-ethylbutanol, and cyclopentylmethanol,

   [Formula 1]

   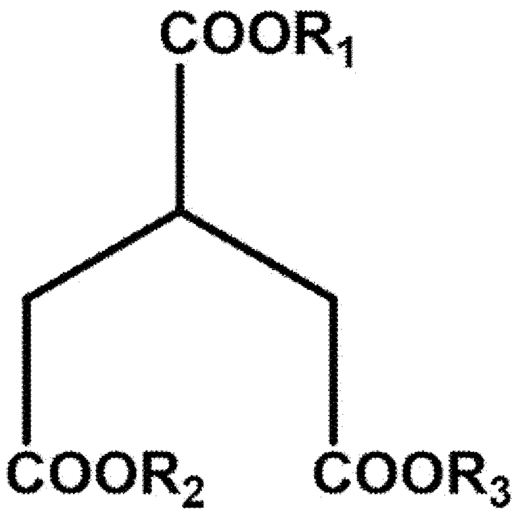

   wherein in Formula 1 above,
   $R_1$ to $R_3$ are each independently an n-hexyl group, a branched hexyl group, or a cyclopentylmethyl group.

2. The plasticizer composition of claim 1, wherein the isomer mixture of hexyl alcohol has a degree of branching of 2.0 or less.

3. The plasticizer composition of claim 2, wherein the isomer mixture of hexyl alcohol has a degree of branching of 1.5 or less.

4. The plasticizer composition of claim 1, wherein the isomer mixture of hexyl alcohol comprises 1-hexanol, 2-methylpentanol, and 3-methylpentanol.

5. The plasticizer composition of claim 1, wherein the isomer mixture of hexyl alcohol comprises a branched alcohol

in an amount of 40 parts by weight or more with respect to 100 parts by weight of the isomer mixture.

**6.** The plasticizer composition of claim 1, wherein the isomer mixture of hexyl alcohol comprises a branched alcohol in an amount of 50 parts by weight to 95 parts by weight with respect to 100 parts by weight of the isomer mixture.

**7.** The plasticizer composition of claim 1, wherein the isomer mixture of hexyl alcohol comprises 1-hexanol in an amount of 40 parts by weight or less with respect to 100 parts by weight of the isomer mixture.

**8.** The plasticizer composition of claim 1, wherein the isomer mixture of hexyl alcohol comprises 1-hexanol, 2-methylpentanol, 3-methylpentanol, and cyclopentylmethanol.

**9.** The plasticizer composition of claim 8, wherein the isomer mixture of hexyl alcohol comprises cyclopentylmethanol in an amount of 20 parts by weight or less with respect to 100 parts by weight of the isomer mixture.

**10.** A resin composition comprising:
a resin in an amount of 100 parts by weight; and the plasticizer composition of claim 1 in an amount of 5 parts by weight to 150 parts by weight.

**11.** The resin composition of claim 10, wherein the resin is one or more selected from the group consisting of a straight vinyl chloride polymer, a paste vinyl chloride polymer, an ethylene vinyl acetate copolymer, an ethylene polymer, a propylene polymer, polyketone, polystyrene, polyurethane, polylactic acid, natural rubber, and synthetic rubber.

**INTERNATIONAL SEARCH REPORT**

International application No. **PCT/KR2022/016615**

**A. CLASSIFICATION OF SUBJECT MATTER**

**C08K 5/092**(2006.01)i; **C08K 5/00**(2006.01)i; **C08L 101/00**(2006.01)i

According to International Patent Classification (IPC) or to both national classification and IPC

**B. FIELDS SEARCHED**

Minimum documentation searched (classification system followed by classification symbols)

C08K 5/092(2006.01); C07C 67/02(2006.01); C07C 69/34(2006.01); C07C 69/704(2006.01); C08K 5/00(2006.01); C08K 5/10(2006.01); C08K 5/11(2006.01); C09D 201/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus, Castract), Google & keywords: 프로판 트리카복실레이트(propane tricarboxylate), 핵실 알코올(hexyl alcohol), 이성질체(isomer), 가소제(plasticizer)

**C. DOCUMENTS CONSIDERED TO BE RELEVANT**

| Category* | Citation of document, with indication, where appropriate, of the relevant passages | Relevant to claim No. |
|---|---|---|
| Y | MAGNE, F. C. et al. Plasticizers from aconitic and tricarballylic acids. Industrial and engineering chemistry. 1953, vol. 45, no. 7, pp. 1546-1547. See page 1546; and table 1. | 1-11 |
| Y | KR 10-2021-0014597 A (LG CHEM, LTD.) 09 February 2021 (2021-02-09) See abstract; claims 1-10; and paragraphs [0078] and [0079]. | 1-11 |
| A | JP 2019-515986 A (ASCEND PERFORMANCE MATERIALS OPERATIONS LLC) 13 June 2019 (2019-06-13) See claims 1-33. | 1-11 |
| A | KR 10-2021-0092140 A (LG CHEM, LTD.) 23 July 2021 (2021-07-23) See claims 1-10. | 1-11 |
| A | KR 10-2017-0020282 A (LG CHEM, LTD.) 22 February 2017 (2017-02-22) See claims 1-20. | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 February 2023** | **02 February 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2022/016615**

**C. DOCUMENTS CONSIDERED TO BE RELEVANT**

| Category* | Citation of document, with indication, where appropriate, of the relevant passages | Relevant to claim No. |
|---|---|---|
| A | KR 10-1952338 B1 (LG CHEM, LTD.) 26 February 2019 (2019-02-26) See claims 1-7. | 1-11 |

Form PCT/ISA/210 (second sheet) (July 2022)

**INTERNATIONAL SEARCH REPORT**
**Information on patent family members**

International application No.
**PCT/KR2022/016615**

| Patent document cited in search report | Publication date (day/month/year) | Patent family member(s) | Publication date (day/month/year) |
|---|---|---|---|
| KR 10-2021-0014597 A | 09 February 2021 | BR 112021023388 A2 | 04 January 2022 |
| | | CN 113785010 A | 10 December 2021 |
| | | EP 4006091 A1 | 01 June 2022 |
| | | JP 2022-529284 A | 20 June 2022 |
| | | TW 202116722 A | 01 May 2021 |
| | | US 2022-0195143 A1 | 23 June 2022 |
| | | WO 2021-020878 A1 | 04 February 2021 |
| JP 2019-515986 A | 13 June 2019 | CA 3019547 A1 | 12 October 2017 |
| | | CN 109195939 A | 11 January 2019 |
| | | CN 109195939 B | 28 June 2022 |
| | | EP 3440044 A1 | 13 February 2019 |
| | | JP 6937318 B2 | 22 September 2021 |
| | | MX 2018012082 A | 28 March 2019 |
| | | US 11390762 B2 | 19 July 2022 |
| | | US 2020-339826 A1 | 29 October 2020 |
| | | WO 2017-176504 A1 | 12 October 2017 |
| KR 10-2021-0092140 A | 23 July 2021 | CN 113748159 A | 03 December 2021 |
| | | EP 3943540 A1 | 26 January 2022 |
| | | TW 202136200 A | 01 October 2021 |
| | | US 2022-0220281 A1 | 14 July 2022 |
| | | WO 2021-145642 A1 | 22 July 2021 |
| KR 10-2017-0020282 A | 22 February 2017 | CN 106795325 A | 31 May 2017 |
| | | CN 111732756 A | 02 October 2020 |
| | | CN 111732757 A | 02 October 2020 |
| | | CN 111777796 A | 16 October 2020 |
| | | EP 3130631 A1 | 15 February 2017 |
| | | EP 3130631 B1 | 01 April 2020 |
| | | EP 3670592 A1 | 24 June 2020 |
| | | EP 3670593 A1 | 24 June 2020 |
| | | KR 10-1674317 B1 | 08 November 2016 |
| | | KR 10-2004732 B1 | 29 July 2019 |
| | | KR 10-2016-0130363 A | 11 November 2016 |
| | | KR 10-2079234 B1 | 19 February 2020 |
| | | KR 10-2197487 B1 | 31 December 2020 |
| | | TW 201700568 A | 01 January 2017 |
| | | TW I617605 B | 11 March 2018 |
| | | US 11359071 B2 | 14 June 2022 |
| | | US 2017-0081501 A1 | 23 March 2017 |
| | | US 2022-0251336 A1 | 11 August 2022 |
| | | US 2022-0325070 A1 | 13 October 2022 |
| | | WO 2016-129876 A1 | 18 August 2016 |
| KR 10-1952338 B1 | 26 February 2019 | CN 109563309 A | 02 April 2019 |
| | | CN 109563309 B | 03 November 2020 |
| | | CN 109563310 A | 02 April 2019 |
| | | CN 109563310 B | 06 April 2021 |
| | | EP 3476890 A1 | 01 May 2019 |
| | | EP 3476890 B1 | 01 April 2020 |
| | | EP 3476891 A1 | 01 May 2019 |
| | | EP 3476891 B1 | 28 September 2022 |
| | | KR 10-1982191 B1 | 24 May 2019 |

Form PCT/ISA/210 (patent family annex) (July 2022)

**INTERNATIONAL SEARCH REPORT**
**Information on patent family members**

International application No.
**PCT/KR2022/016615**

| Patent document cited in search report | Publication date (day/month/year) | Patent family member(s) | Publication date (day/month/year) |
|---|---|---|---|
| | | TW 201835187 A | 01 October 2018 |
| | | TW 201835188 A | 01 October 2018 |
| | | TW I733984 B | 21 July 2021 |
| | | TW I748055 B | 01 December 2021 |
| | | US 11130852 B2 | 28 September 2021 |
| | | US 11499030 B2 | 15 November 2022 |
| | | US 2019-0211183 A1 | 11 July 2019 |
| | | US 2021-0070964 A1 | 11 March 2021 |
| | | WO 2018-147689 A1 | 16 August 2018 |
| | | WO 2018-147690 A1 | 16 August 2018 |

Form PCT/ISA/210 (patent family annex) (July 2022)